# EUROPÄISCHE PATENTANMELDUNG

(11) **EP 1 059 153 A2**
(43) Veröffentlichungstag der Anmeldung: **13.12.2000**
(21) Anmeldenummer: 00109165.1
(22) Anmeldetag: 08.05.2000
(51) Int. Cl.: B29C 33/00, B29C 43/36, A61F 2/28

(54) **Fertigungsform für Knochenersatzimplantate**

(30) Priorität: 09.06.1999 DE 19926270
(71) Anmelder: MERCK PATENT GmbH, 64293 Darmstadt (DE)
(72) Erfinder: Dard, Michael, Dr., 64342 Seeheim-Jugenheim (DE); Chryssikopoulos, Spyros, Dr., 17121 Athen (GR)

(57) **Zusammenfassung**

Eine Fertigungsform zur industriellen und intraoperativen Herstellung von implantierfähigen Fertigstücken aus Knochenersatzmaterialien weist eine Trägerplatte (1), einen Formrahmen (2) mit einer zentralen Aussparung (5) für die Aufnahme des zu implantierenden Knochenersatzmaterials und eine Deckelplatte (3) auf. Diese sind während des Abbindevorgangs des ursprünglich pastösen Knochenersatzmaterials paßgenau übereinander angeordnet. Die Form des herstellbaren Knochenersatzimplantats wird durch einen ebenen oder gekrümmten Formrahmen (2) mit einer im wesentlichen beispielsweise kreisförmigen, rechteckigen oder dreieckigen Aussparung (5) bestimmt.

## Beschreibung

Die Erfindung betrifft eine Fertigungsform zur industriellen und intraoperativen Herstellung von implantierfähigen Fertigstücken aus Knochenersatzmaterialien. Sie soll Verwendung bei Knochenoperationen finden, in denen die Implantation eines Knochenersatzmaterials erforderlich ist, insbesondere in der orthopädischen Chirurgie, der Mund-Zahn-Kiefer-Gesichts-Chirurgie und der Neurochirugie.

Ein häufig verwendetes Knochenersatzmaterial ist beispielsweise hydrolytischer Calciumphosphatzement. Das aus einer Flüssigkeit und einem Pulver zusammen gemischte Knochenersatzmaterial wird in pastösem Zustand mit einem Spatel in den zu behandelnden Knochendefekt eingeführt.

Der Nachteil der pastösen Konsistenz hydrolytischer Calciumphosphatzemente zu Beginn der Anwendung ist, daß diese Knochenersatzmaterialien nur bei mehrwandigen Knochendefekten erfolgreich eingesetzt werden können, da während der Abbindephase ein Volumenschwund des in den Knochendefekt eingebrachten Knochenersatzmaterials auftritt. Um Deckknochendefekte oder beispielsweise Schäden der Kortikalis zu rekonstruieren (z.B. kranial oder diaphysär), braucht man dagegen ein formstabiles Knochenersatzimplantat mit einer Primärstabilität. Dieses Knochenersatzimplantat fungiert dann als zusätzliche "Kortikalis". Um eine Adaption des Knochenersatzimplantats mit dem umgebenden Knochenbereich zu ermöglichen ist es vorteilhaft, sowohl für das Knochenersatzimplantat als auch das Verbundmittel ein Material mit der selben chemischen Struktur zu verwenden. Die Herstellung eines vorgefertigten Knochenersatzimplantats und die Adaption muß also mit dem selben Calciumphosphatzement erfolgen.

Die Resorptionsfähigkeit eines schon abgebundenen Calciumphosphatzements ist geringer als die eines noch pastösen. Der ungewollte Volumenschwund erfolgt überwiegend bei der pastösen Einführung des Materials. Dabei variiert der Volumenschwund je nach Durchblutungsgrad oder Schwellungsbereitschaft des Gewebes. Hohe Durchblutung oder Schwellungsbereitschaft führen dazu, daß während der Abbindephase des Calciumphosphatzements die Paste durch die Körperflüssigkeiten (Blut, Gewebeflüssigkeit der Weichteile, Ödembildung etc.) noch zusätzlich an den Kontaktstellen zu den Wundrändern verdünnt wird. Die Unterminierung des eingeführten Materials oder die Verlängerung seiner Abbindephase sind die Folge. Ein zusätzlicher Volumenschwund tritt als Folgeerscheinung ein. Es kommt zu einer Lageveränderung, einer Auflösung oder sogar zur Abstoßung des implantierten Knochenersatzmaterials infolge des Volumenschwundes.

Es ist bereits bekannt (US 5 397 361 A), ein Knochenersatzimplantat herzustellen, indem zunächst mit einem Abdruckmaterial eine Negativform des einem Patienten entnommenen Knochenstücks gefertigt und anschließend mittels dieser Form aus Knochenersatzmaterial ein Implantat hergestellt wird, das die gleiche Form wie das entnommene Knochenstück aufweist. Bei diesem Verfahren wird eine zweiteilige Form zur Herstellung des Implantats benutzt. Da die zweiteilige Form als Negativform des dem Patienten entnommenen Knochenstücks für jede Anwendung gesondert hergestellt werden muß, kann sie als nur einmal genau für diesen Patienten verwendbare Form nicht für weitere Implantate genutzt werden.

Eine bekannte Fertigungsform der eingangs genannten Gattung zur Herstellung eines für die Ersetzung des Hüftgelenks vorgesehenen Knochenimplantats (US 5 645 594 A) bildet eine verschließbare Negativform, die dem Anfertigen vieler identischer, sphärisch gekrümmter Knochenersatzimplantate dient. Eine Anpassung der Negativform an verschiedene anatomische Gegebenheiten unterschiedlicher Indikationsgebiete ist ebenso wie die Herstellung nur eines kleinen Teilstücks aus Knochenersatzmaterial nicht möglich.

Aufgabe der vorliegenden Erfindung ist es daher, eine Fertigungsform für Knochenersatzimplantate so auszugestalten, daß mit geringem Aufwand eine Umstellung auf eine möglichst große Formenvielfalt verschiedener Fertigstücke ermöglicht wird.

Diese Aufgabe wird erfindungsgemäß dadurch gelöst, daß die Fertigungsform eine Trägerplatte, einen Formrahmen mit einer zentralen Aussparung für die Aufnahme des zu implantierenden Knochenersatzmaterials und eine Deckelplatte aufweist, die paßgenau übereinander verbindbar sind.

Der erfindungsgemäße Gegenstand weist eine Trägerplatte mit einer entsprechend der anatomischen Gegebenheiten des vorgesehenen Indikationsgebietes, wie beispielsweise dem Kieferhöhlen-Boden, dem Orbitaboden, der Schädelkalotte oder einer Gelenkgrube (z.B. Acetabulum), geformten Oberfläche auf. Das zu implantierende Knochenersatzmaterial wird auf die Trägerplatte aufgetragen, um dort abzubinden und zu einer endgültigen Form verarbeitet zu werden. Die maximalen Abmaße des Implantats werden durch die Aussparungen unterschiedlicher Formrahmen sowie die abschließende Deckplatte vorgegeben. Für eine genaue Anpassung an den zu behandelnden Knochendefekt kann das vorgefertigte Knochenersatzimplantat nach dem Abbinden beispielsweise mit sterilisierbaren, diamantierten Fräsern oder Trennscheiben exakt zugeschnitten werden.

Im Rahmen einer industriellen Fertigung kann das Fertigstück vom Hersteller sterilisiert (z.B. mittels γ-Strahlen) und abgepackt werden; der Chirurg bekommt das gewünschte Fertigstück geliefert. Das Implantat aus Knochenersatzmaterial kann aber auch vom Chirurgen intraoperativ hergestellt und sofort implantiert werden. Der wesentliche Vorteil gegenüber der industriellen Herstellung liegt in einer beträchtlichen Zeit- und Kostenersparnis.

Gerade im Hinblick auf den intraoperativen Einsatz wird eine für jeden Einzelfall notwendige Anpassung des hergestellten Knochenersatzimplantats mit vergleichsweise wenig Aufwand ermöglicht, so daß die Anpassung schnell und unkompliziert vorgenommen werden kann. Mehrere verschiedene Fertigungsformen können mit geringem fertigungstechnischem Aufwand hergestellt werden, da nur jeweils der Formrahmen, nicht aber die gesamte Fertigungsform anzufertigen ist.

Als verwendbare Knochenersatzmaterialien sind alle bekannten Calciumphosphatzemente, aber auch Kunststoffzemente oder PMMA denkbar. Je nach Verarbeitungsmöglichkeiten des zu implantierenden Knochenersatzmaterials kann die Fertigungsform auf die notwendige Bearbeitungstemperatur abgekühlt oder erwärmt werden. Zum Beispiel erfolgt die Erwärmung der Fertigungsform und der Mischflüssigkeit des hydrolytischen Calciumphosphatzements in einem Brutschrank (oder Wasserbad o.ä.) mit Temperaturanzeige, die Abkühlung im Kühlschrank.

Durch die Verwendung der so hergestellten Fertigstücke bzw. Implantate ist das Ausbleiben einer Flüssigkeitsaufnahme durch die gesättigte, abgebundene chemische Struktur des Fertigstückes, die stabile Formgebung des Implantats, die schnelle Adaption des Implantats an die Defektgrenzen und die Reduktion der Resorptionsflächen des implantierten Materials gewährleistet.

Einer vorteilhaften Ausgestaltung des Erfindungsgedankens zufolge ist vorgesehen, daß der Formrahmen eine Krümmung um eine Krümmungsachse aufweist und die Trägerplatte eine daran angepaßte gekrümmte Oberseite sowie die Deckelplatte eine daran angepaßte gekrümmte Unterseite besitzt. Der Krümmungsradius kann dabei im Hinblick auf ein bevorzugtes Anwendungsgebiet vorgegeben werden, so daß die aufwendige Anpassung des vorgefertigten Knochenersatzimplantats an den im Einzelfall zu behandelnden Knochendefekt weitgehend reduziert wird.

Einer besonders vorteilhaften Ausführung des Erfindungsgedankens zufolge ist vorgesehen, daß der Formrahmen je nach Anwendung mit einer im wesentlichen runden, quadratischen oder dreieckigen zentralen Aussparung versehen ist. Durch die Gestaltung des im Einzelfall verwendeten Formrahmens kann das vorgefertigte Knochenersatzimplantat weitgehend an den jeweils zu behandelnden Knochendefekt angepasst werden. Darüberhinaus sind eventuell nur noch geringe Modifikationen des Knochenersatzimplantats notwendig.

Gemäß einer vorteilhaften Ausgestaltung des Erfindungsgedankens ist vorgesehen, daß die Trägerplatte, der Formrahmen und die Deckenplatte aus sterilisierbarem Material bestehen. Eine intraoperative Verwendung der Fertigungsform in sterilen Bereichen wie beispielsweise im Operationsraum ist dadurch möglich.

Vorzugsweise ist vorgesehen, daß die Fertigungsform aus Aluminium oder Edelstahl besteht. Eine absolute Hochglanzpolitur der Kontaktflächen gewährleistet die einfache Entnahme des Fertigteils und eine vereinfachte Reinigung sowohl durch die Helferin oder OP-Schwester als auch bei der industriellen Herstellung.

Weitere vorteilhafte Ausgestaltungen des Erfindungsgedankens sind Gegenstand weiterer Unteransprüche.

Nachfolgend wird ein Ausführungsbeispiel erläutert, welches in der Zeichnung dargestellt ist. Es zeigt:
Fig. 1 eine Ansicht einer dreiteiligen Fertigungsform in auseinandergezogener Darstellung,
Fig. 2 einen Schnitt durch die dreiteilige Fertigungsform,
Fig. 3 eine Ansicht eines auf eine Trägerplatte gelegten sphärisch gekrümmten Formrahmens mit einer konzentrischen, kreisrunden Aussparung,
Fig. 4 einen Schnitt entlang der Linie IV-IV in Fig.3,
Fig. 5 eine Ansicht des auf die Trägerplatte gelegten sphärisch gekrümmten Formrahmens mit einer rechteckigen Aussparung und
Fig. 6 eine Ansicht des auf die Trägerplatte gelegten sphärisch gekrümmten Formrahmens mit einer dreieckigen Aussparung.

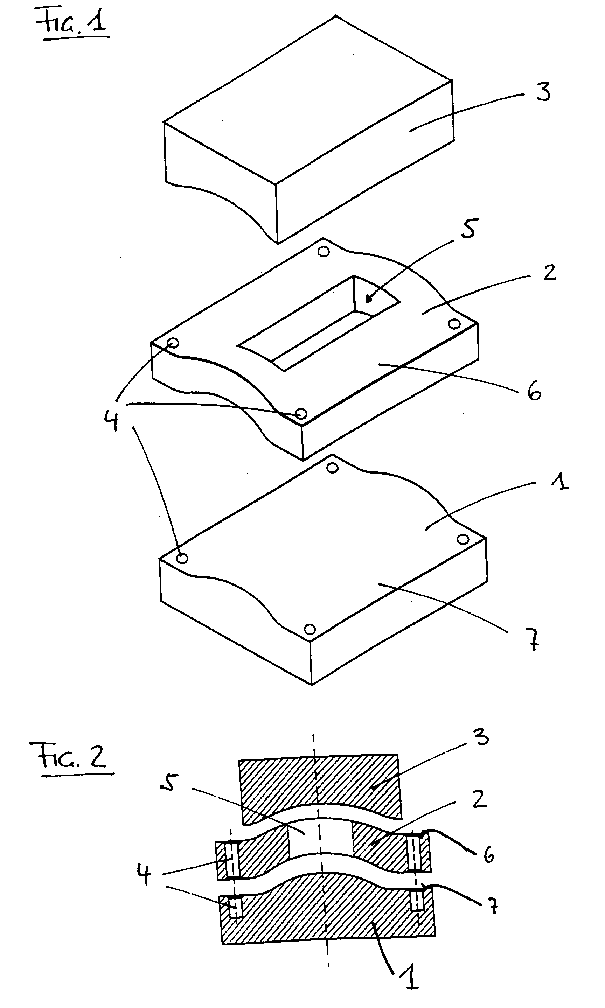

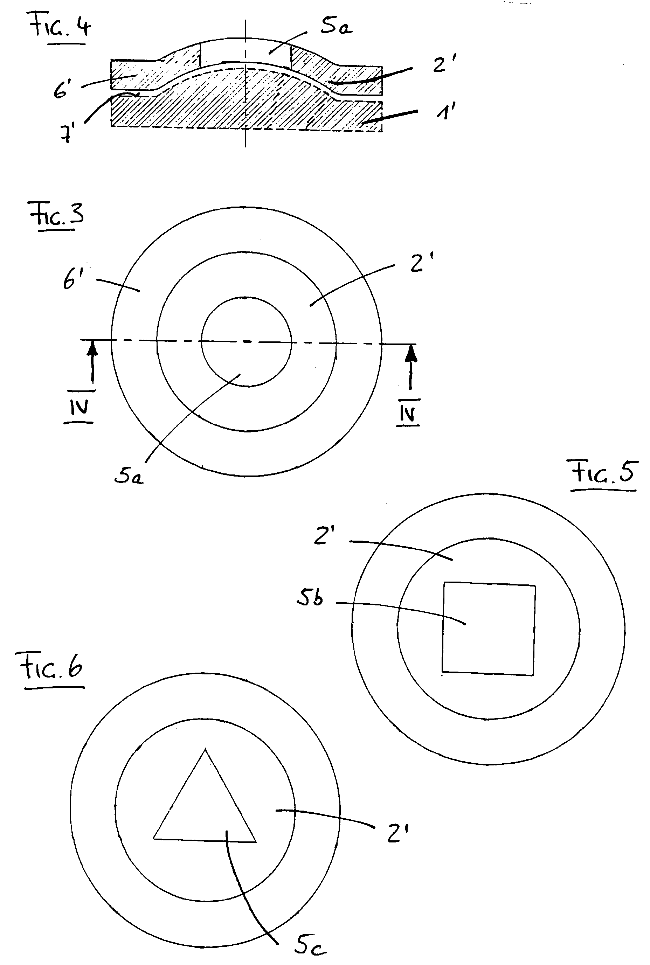

Die in den Figuren 1 und 2 gezeigte Fertigungsform besteht aus einer Trägerplatte 1, einem Formrahmen 2 sowie einer Deckenplatte 3. Die Oberfläche der Trägerplatte 1 weist eine in einer Richtung konvexe Krümmung um eine Krümmungsachse auf, die an eine konkave Krümmung der Unterseite des Formrahmen 2 angepasst ist. Eine konstante Dicke des Formrahmens 2 wird durch eine identische gekrümmte Ausführung der Oberseite des Formrahmens 2 sowie der Unterseite der Deckenplatte 3 erreicht. Die Trägerplatte 1 und der Formrahmen 2 weisen in den Ecken befindliche Bohrungen 4 auf. Diese sind für eine feste Verbindung von der Trägerplatte 1 und dem Formrahmen 2 beispielsweise durch einsetzbare Stifte vorgesehen.

Statt der Verbindung durch einsetzbare Stifte können auch Bügel oder in entsprechend angepaßte Ausnehmungen der Trägerplatte 1 oder des Formrahmens 2 eingreifende Ausformungen des jeweils anderen Teils realisiert sein.

Der Formrahmen 2 weist eine zentral angeordnete, rechteckige Aussparung 5 auf. Nachdem der Formrahmen 2 auf der Trägerplatte 1 aufgesetzt wurde, kann das pastöse Knochenersatzmaterial in die Aussparung 5 gefüllt werden und dort abbinden. Die Krümmung der Trägerplatte 1 sowie des Formrahmens 2 entspricht im Bereich der Aussparung 5 weitgehend der gewünschten Krümmung des beispielsweise im Kieferhöhlenboden einzusetzenden Knochenimplantats. Die Aussparung 5 kann durch Aufsetzen der Deckenplatte 3 während des Abbindevorgangs abgeschlossen werden. Damit werden Überschüsse des Knochenersatzmaterials entfernt. Nach dem Abbinden kann man die Deckelplatte 3 problemlos entfernen und das Fertigstück entnehmen.

Der Formrahmen 2 weist an den beiden parallel zur Krümmungsachse verlaufenden Seiten je einen flachen Rand 6 auf, der auf einer paßgenau ausgeformten, ebenen Auflagefläche 7 der Trägerplatte 1 aufliegt.

Für bestimmte Anwendungen ist es denkbar, die Anordnung von der Trägerplatte 1, dem Formrahmen 2 und der Deckenplatte 3 zu vertauschen, so daß sich die Deckenplatte 3 unter dem Formrahmen 2 und der oben aufliegenden Trägerplatte 1 befindet.

Dadurch, daß die Elemente in der Mitte und längs gekrümmt sind, sind die einzelnen Teile automatisch gegen seitliches Verschieben gesichert. Die Abmaße sind so gewählt, daß die Dicke des Fertigstücks 8 mm nicht unterschreitet. Die Aussparung 5 in dem Formrahmen 2 für die Herstellung des Fertigstückes ist 15 mm breit und 40 mm lang. Die im Einzelfall für eine Anwendung gewünschte Länge des Fertigstückes kann man durch Bearbeitung mit sterilen Fräsen oder Trennscheiben erreichen.

Auf die Trägerplatte 1 wird z.B. der Calciumphosphatzement nach dem Anmischen aufgetragen. Um eine schnelle Abbindung zu gewährleisten, wird die erfindungsgemäße Vorrichtung entsprechend der Herstellerangaben auf die notwendige Temperatur gebracht.

Nachdem das Knochenersatzimplantat intraoperativ fertiggestellt wurde, kommt es zu seinem Einsatz beispielsweise bei der kombinierten Sinus-Lift-Operation mit Implantation eines oder mehrerer Zahnimplantate. Ein Knochenfenster (Breite etwa 5-10 mm; Länge etwa 10-40 mm) wird vestibulär an die Kieferhöhle gesetzt. Die Fensterlänge wird im voraus röntgenographisch bestimmt. Daraus resultiert auch die Länge des z.B. aus Calciumphosphatzement hergestellten Fertigstücks. Das Fertigstück wird zwischen Kieferhöhlenschleimhaut und Knochen geschoben. Die Bohrung für die Implantate kann jetzt erfolgen. Durch das Fertigstück ist die Kieferhöhlenschleimhaut vor Perforationen geschützt. Beim Eindrehen der Implantate fungiert das Fertigstück als Elevatorium für die Schleimhaut und hebt diese bis zum Anschlag des Implantatgewindes an. Der gewonnene Hohlraum wird mit dem gleichen Knochenersatzmaterial des Fertigstückes in pastöser Form oder Konsistenz jetzt aufgefüllt. Die Reste werden nach der ersten Abbindephase entfernt. Die Wunde wird primär verschlossen.

Diese Operationstechnik ist sinnvoll, wenn der Verlust der vestibulären Knochenwand zur Destabilisierung des Operationsfeldes und zur Gefährdung der Primärstabilität der Implantate führen könnte.

Im Falle einer Kieferkammdicke zwischen 3 und 5 mm kann man sogar mit zwei Fertigstücken sowohl die Kieferhöhlenschleimhaut elevieren als auch ein zusätzliches Lager für die Implantate schaffen. Die Entnahme eines autologen Knochentransplantates zur Unterstützung des Kieferkamms wird somit überflüssig.

In den Figuren 3 und 4 ist ein auf einer Trägerplatte 1' aufgelegter Formrahmen 2' dargestellt, der eine konzentrische, sphärische Krümmung aufweist. Der Formrahmen 2' hat eine ebenfalls konzentrisch positionierte, kreisförmige Aussparung 5a, die zur Aufnahme des zu Beginn noch pastösen Knochenersatzmaterials vorgesehen ist. Der Formrahmen 2' weist einen umlaufenden, flachen Rand 6' auf, der auf einer paßgenau ausgeformten Auflagefläche 7' der Trägerplatte 1' liegt. Durch den umlaufenden, flachen Rand 6' ist eine ungewollte Verschiebung des Formrahmens 2' relativ zur Trägerplatte 1' unmöglich. Zusätzliche Vorrichtungen zum sicheren Befestigen des Formrahmens 2' auf der Trägerplatte 1' sind deshalb nicht notwendig, können aber für bestimmte Anwendungen vorteilhaft sein. Auf den Formrahmen 2' kann bei Bedarf eine nicht in der Zeichnung dargestellte, entsprechend der sphärischen Krümmung sowie dem umlaufenden, flachen Rand 6' geformte Deckelplatte aufgelegt werden.

In den Figuren 5 und 6 sind Ansichten weiterer Ausführungsbeispiele des Formrahmens 2' gezeigt. Allen Ausführungen gemeinsam ist, daß der Formrahmen 2' eine im wesentlichen konvexe Krümmung aufweist und die Trägerplatte 1' eine daran angepaßte Oberseite sowie die (in den Figuren nicht dargestellte) Deckelplatte 3' eine daran angepaßte Unterseite besitzt. Der in Fig. 5 gezeigte Formrahmen 2' weist dabei eine rechteckige Aussparung 5b, bzw. der in Fig. 6 dargestellte Formrahmen 2' eine dreieckige Aussparung 5c auf.

Für die intraoperative Anwendung der Fertigungsform ist denkbar, daß der Formrahmen 2' je nach Anwendung mit einer im wesentlichen runden, quadratischen oder dreieckigen zentralen Aussparung 5a, 5b, 5c versehen ist. Das Knochenersatzimplantat kann mit Hilfe von sterilen Fräsen intraoperativ entsprechend dem Implantatbett geformt und ausgearbeitet werden.

Das so hergestellte Fertigstück kommt beispielsweise zum Einsatz bei der infraorbitalen Implantation zur Vergrößerung des Orbitabodens. Bei Exophthalmus z.B. kann dadurch die Augenstellung erheblich verbessert werden. Je nach erforderlicher Form (Kalotte, Dreieck oder Quader) wird das entsprechende Fertigstück intraoperativ verarbeitet, wobei die Größe mit Hilfe der Computertomographie im voraus bestimmt wird.

Die Orbitahöhle wird meist unter dem unteren Augenlid eröffnet und durch Excochleation des Auges dargestellt. Meist im Bereich der Sutura infraorbitalis wird der knöcherne Infraorbitaboden gespreizt und das Fertigstück eingesetzt. Die angrenzenden Flächen zu dem Knochen bzw. der Spalt zwischen Knochen und Fertigstück werden mit der pastösen Form des implantierten Materials aufgefüllt. Die Reste werden nach der ersten Abbindephase entfernt. In das Fertigstück kann vom Chirurgen eine Aussparung für den Nervus infraorbitalis eingeschliffen werden. Eine zusätzliche Stabilisierung der Knochenstücke gegeneinander z.B. mit einer Osteosyntheseplatte entfällt bei guter Verkeilung des implantierten Fertigstücks. Die Abdeckung des Implantatbettes mit zwei resorbierbaren Kollagenmembranen kann zu einer schnelleren Adaption des Materials an den Knochen beitragen.

## Patentansprüche

1. Fertigungsform zur industriellen und intraoperativen Herstellung von implantierfähigen Fertigstücken aus Knochenersatzmaterialien,
dadurch gekennzeichnet,
daß die Fertigungsform eine Trägerplatte (1,1'), einen Formrahmen (2,2') mit einer zentralen Aussparung (5,5a,5b,5c) für die Aufnahme des zu implantierenden Knochenersatzmaterials und eine Deckelplatte (3) aufweist, die paßgenau übereinander verbindbar sind.

2. Fertigungsform nach Anspruch 1,
dadurch gekennzeichnet,
daß der Formrahmen (2) eine Krümmung um eine Krummungsachse aufweist und die Trägerplatte (1) eine daran angepaßte gekrümmte Oberseite sowie die Deckelplatte (3) eine daran angepaßte gekrümmte Unterseite besitzt.

3. Fertigungsform nach Anspruch 1,
dadurch gekennzeichnet,
daß der Formrahmen (2') eine sphärische Krümmung aufweist und die Trägerplatte (1') eine daran angepaßte Oberseite sowie die Deckelplatte (3') eine daran angepaßte Unterseite besitzt.

4. Fertigungsform nach Anspruch 2 oder 3,
dadurch gekennzeichnet,
daß der Formrahmen (2,2') eine im wesentlichen konvexe Krümmung aufweist und die Trägerplatte (1,1') eine daran angepaßte Oberseite sowie die Deckelplatte (3,3') eine daran angepaßte Unterseite besitzt.

5. Fertigungsform nach Anspruch 2 oder 3,
dadurch gekennzeichnet,
daß die Trägerplatte (1,1') und der Formrahmen (2,2') gegen horizontales Verschieben gesichert sind.

6. Fertigungsform nach Anspruch 1,
dadurch gekennzeichnet,
daß die Trägerplatte (1,1') und der Formrahmen (2,2') Ausnehmungen (4) aufweisen und mit paßgenau in diese Ausnehmungen (4) einsetzbaren Stiften gegen horizontales Verschieben gesichert sind.

7. Fertigungsform nach Anspruch 2,
dadurch gekennzeichnet,
daß der Formrahmen (2) und die Deckelplatte (3) entlang der Krümmungsachse gegeneinander verschiebbar sind.

8. Fertigungsform nach Anspruch 1,
dadurch gekennzeichnet,
daß die Fertigungsform aus sterilisierbarem Material besteht.

9. Fertigungsform nach Anspruch 8,
dadurch gekennzeichnet,
daß die Fertigungsform aus Aluminium oder Edelstahl besteht.

10. Fertigungsform nach Anspruch 1,
dadurch gekennzeichnet,
daß der Formrahmen (2,2') mit einer im wesentlichen runden, quadratischen oder dreieckigen zentralen Aussparung (5,5a,5b,5c) versehen ist.
